(19) Europäisches Patentamt
European Patent Office
Office européen des brevets

(11) Veröffentlichungsnummer: **0 394 907**
**A1**

(12) **EUROPÄISCHE PATENTANMELDUNG**

(21) Anmeldenummer: **90107637.2**

(51) Int. Cl.5: **C07H 17/04, C07H 15/06**

(22) Anmeldetag: **23.04.90**

(30) Priorität: **22.04.89 DE 3913326**

(43) Veröffentlichungstag der Anmeldung:
**31.10.90 Patentblatt 90/44**

(84) Benannte Vertragsstaaten:
**AT BE CH DE DK ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Kolar, Cenek, Dr.**
**Deutschhausstrasse 20**
**D-3550 Marburg(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Verfahren zur Herstellung von Etoposiden.**

(57) Die Erfindung betrifft ein Verfahren zur Herstellung eines beta-Glucopyranose-Derivates der Formel I

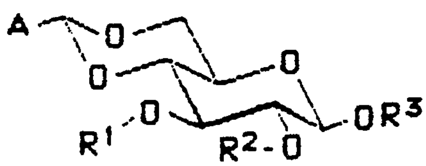

I

worin
$R^1$ und $R^2$ H oder eine Acyl-Schutzgruppe und
$R^3$ H, Benzyl oder ein Rest der Formel II

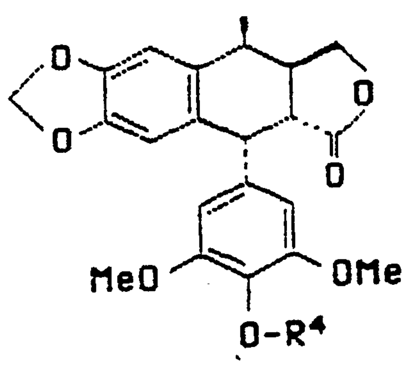

II

mit $R^4$ H oder eine Benzyloxycarbonyl- oder Chloracetyl-Schutzgruppe und
A $C_1$-$C_4$-Alkyl darstellt, dadurch gekennzeichnet, daß man in einem Benzylglucopyranosid der Formel I, worin
$R^1$ und $R^2$ eine Acyl-Schutzgruppe,
$R^3$ eine Benzyl-Schutzgruppe und

EP 0 394 907 A1

A $C_1$-$C_4$-Alkyl sind,
die Benzylgruppe hydrogenolytisch in der Gegenwart eines Hydrierungskatalysators und eines organischen Lösungsmittels abspaltet, wobei ein in der beta-Hydroxy-Form vorliegendes Glucopyranose- Derivat der Formel I, worin
$R^1$, $R^2$ und A ihre Bedeutung beibehalten und
$R^3$ ein Wasserstoffatom ist, gebildet wird, anschließend diese Glycosidierungskomponente mit einem epi-Podophyllotoxin-Derivat der Formel III

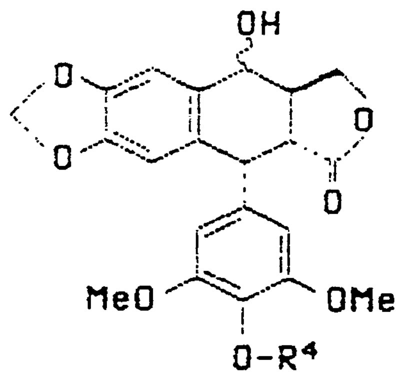

III

worin
$R^4$ Benzyloxycarbonyl- oder Chloracetyl-Schutzgruppe ist, in der Gegenwart eines Promotors wie $BF_3$xEther oder eines Trifluormethansulfonsäure-tri-($C_1$-$C_4$)-alkylsilylesters und eines organischen Lösungsmittels, gegebenenfalls in der Präsenz eines Trockenmittels bei -30 °C bis 0 °C umsetzt, wobei ein beta-Glycosid der Formel I, worin $R^1$, $R^2$, $R^4$ und A ihre Bedeutung beibehalten und $R^3$ einen Rest der Formel II darstellt, entsteht, und in einem gebildeten Produkt die Benzyloxycarbonyl-Schutzgruppe hydrogenolytisch und die Acyl-Schutzgruppe mittels basischer Ionenaustauscher in der Gegenwart eines polaren Lösungmittels abspaltet, wobei ein Produkt der Formel I entsteht, worin
$R^1$ und $R^2$ Wasserstoff,
$R^3$ ein Rest der Formel II mit $R^4$ gleich Wasserstoff und
A $C_1$-$C_4$-Alkyl sind.

## Verfahren zur Herstellung von Etoposiden

Die Erfindung bezieht sich auf ein Verfahren zur Herstellung von 4-O-(4,6-O-Alkyliden-beta-D-glucopyranosyl)-4′-O-demethyl-4-epi-podophyllotoxinen, die als Etoposide bezeichnet werden, besonders von Etoposid (VP-16), das aufgrund seiner zytostatischen Wirksamkeit für die Behandlung von Krebserkrankungen geeignet ist. Die Erfindung bezieht sich besonders auf ein Verfahren zur Herstellung von 4,6-O-Alkyliden-2,3-di-O-acyl-beta-D-glucopyranosen, die als Glycosyl-Donatoren zur Synthese von 4-O-beta-D-Glucopyranosyl-epi-podophyllotoxinen dienen sowie auf ein Verfahren zur Deacylierung der Glycosidzwischenprodukte.

Etoposid (VP-16) und das strukturell nah verwandte Teniposid (VM-26) sind als Arzneimittel eingeführt. Sie sind besonders wertvoll zur Behandlung von kleinzelligem Lungenkrebs und Testikulum-Krebs.

Die Herstellung, Wirkung sowie klinische Anwendung der Etoposide wurde von T.W. Doyle in "Etoposide (VP-16) -current status and new developments (Editor. B.F. Issell, F.M. Muggia and S.K. Carter; Academic Press, 1984, S. 15-32) beschrieben.

Etoposide werden üblicherweise ausgehend von einem funktionalisierten Glucopyranose-Baustein und einem 4′-O-geschützten epi-Podophyllotoxin-Derivat hergestellt.

Die Synthese von Etoposiden ausgehend von den Glycosyl-Donoren 2,3-Di-O-acetyl-4,6-O-ethyliden-D-glucose oder 2,3-Di-O-halogenacetyl-4,6-O-ethyliden-D-glucose wurde in dem Schweizer Patent No. 514 578 (GB 823,068) oder EP O 111 058 beschrieben. Die dort beschriebene Herstellung dieser Donoren, die aus den 1-O-Benzyloxycarbonyl-beta-glucose-Vorprodukten gewonnen werden, ist kostspielig und wegen der Anwendung von Benzylchloroformiat als Reagenz gesundheitsgefährdend. Außerdem müssen diese in der beta-Form vorliegenden Vorprodukte aus einem alpha,beta-Gemisch isoliert werden, da nur die beta-Form die für die Glycosidsynthese erforderlichen beta-Hydroxyglucopyranose-Donoren liefert.

In EP O 226 202 A2 ist die Herstellung von Etoposiden unter Anwendung von 1-O-Trialkylzinn-Glucose-Donoren beschrieben.

Aufgrund der chemischen Instabilität des Podophyllotoxin-Aglycons verläuft die konventionelle Entblokkierung der Etoposid-Vorprodukte unter Bildung von Nebenprodukten. Die Deacetylierung, wie in den oben genannten Patentanmeldungen beschrieben, verläuft mittels Zink (II)-Salzkatalysierter Methanolyse. Als Nebenprodukte treten hier der entsprechende, durch die Öffnung des Lactonringes gebildete Hydroxysäuremethylester sowie eine Pikroverbindung auf, die bis etwa 30 % des Reaktionsproduktes ausmachen.

Aufgrund der beschriebenen Nachteile gibt es einen Bedarf an einem einfachen kostengünstigen Verfahren zur Herstellung von Etoposiden.

Es wurde überraschenderweise festgestellt, daß die Hydrogenolyse von Benzyl-2,3-di-O-acyl-4,6-O-ethyliden-beta-D-glucopyranosid den Glycosyl-Donor unter Erhalt der beta-Konfiguration liefert. Da bei dieser Reaktion die alpha-Hydroxy-Form der Glucose-Einheit nicht auftritt, verläuft die folgende Glycosylierung der epi-Podophyllotoxin-Derivate selektiv zu den gewünschten beta-Glycosiden. Weiterhin wurde festgestellt, daß die Deacylierung der Etoposid-Vorprodukte mittels eines basischen Ionenaustauschers ohne Bildung der oben beschriebenen Nebenprodukte verläuft.

Der Erfindung liegt die Aufgabe zugrunde, ein Verfahren zu entwickeln, das die Glycosylierungskomponente 4,6-O-Alkyliden-2,3-di-O-acyl-beta-D-glycopyranose in verbesserten Ausbeuten und in reiner beta-Hydroxy-Form liefert und eine Vereinfachung gegenüber den bekannten Verfahren bedeutet, deren Verwendung zur Glycosylierung von (epi)-Podophyllotoxin-Derivaten sowie die Ausarbeitung einer verbesserten Schutzgruppenchemie an acylierten 4-O-Glucosyl-epi-podophyllotoxinen, die es erlaubt, die Etoposide ohne Bildung der Nebenprodukte herzustellen.

Diese Aufgabe wird gelöst durch das Verfahren zur Herstellung eines beta-Glucopyranose-Derivates der Formel I

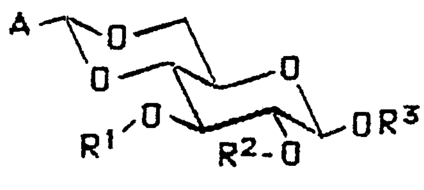

I

worin
$R^1$ und $R^2$ H oder eine Acyl-Schutzgruppe und
$R^3$ H, Benzyl oder der Rest der Formel II

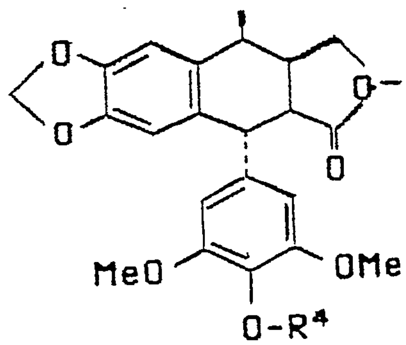

II

mit $R^4$ H oder eine Benzyloxycarbonyl- oder Chloracetyl-Schutzgruppe und A $C_1$-$C_4$-Alkyl darstellt, dadurch gekennzeichnet, daß man in einem Benzylglucopyranosid der Formel I worin
$R^1$ und $R^2$ eine Acyl-Schutzgruppe,
$R^3$ eine Benzyl-Schutzgruppe und
A $C_1$-$C_4$-Alkyl sind,
die Benzylgruppe hydrogenolytisch in der Gegenwart eines Hydrierungskatalysators und eines organischen Lösungsmittels abspaltet, wobei ein in der beta-Hydroxy-Form vorliegendes Glucopyranose-Derivat der Formel I worin $R^1$, $R^2$ und A ihre Bedeutung beibehalten und $R^3$ ein Wasserstoffatom ist, gebildet wird, anschließend diese Glycosidierungskomponente mit einem epi-Podophyllotoxin-Derivat der Formel III

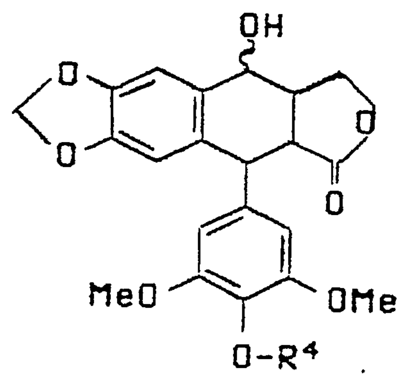

III

worin
$R^4$ Benzyloxycarbonyl- oder Chloracetyl-Schutzgruppe ist, in der Gegenwart eines Promotors, wie vorzugsweise $BF_3$xEther oder eines Trifluormethansulfonsäure-tri-($C_1$-$C_4$)-alkylsilylesters und eines organischen Lösungsmittels, gegebenenfalls in der Präsenz eines Trockenmittels bei -30° C bis 0° C umsetzt, wobei ein beta-Glycosid der Formel I, worin $R^1$, $R^2$, $R^4$ und A ihre Bedeutung beibehalten und $R^3$ einen Rest der Formel II darstellt, entsteht, und in dem gebildeten Produkt die Benzyloxycarbonyl-Schutzgruppe hydrogenolytisch und die Acyl-Schutzgruppe mittels basischer Ionenaustauscher in der Gegenwart eines polaren Lösungmittels abspaltet, wobei ein Produkt der Formel I entsteht, worin $R^1$ und $R^2$ Wasserstoff, $R^3$ ein Rest der Formel II mit $R^4$ gleich Wasserstoff und A $C_1$-$C_4$-Alkyl sind.

Bevorzugt werden nach dem erfindungsgemäßen Verfahren Verbindungen der Formel I hergestellt, worin $R^1$ und $R^2$ H, die Acetyl- oder Chloracetyl-Schutzgruppe, $R^3$ H, Benzyl oder ein Rest der Formel II mit $R^4$ H oder eine Benzyloxycarbonyl- oder Chloracetyl-Schutzgruppe und A Methyl bedeuten.

Im einzelnen geht man dabei wie folgt vor:

Die Ausgangsverbindung Benzyl 2,3-di-O-acyl-4,6-O-alkyliden-beta-D-glucopyranosid kann nach den in der Kohlehydratchemie üblichen Verfahren hergestellt werden. Ausgehend von dem technisch leicht zugänglichen 2,3,4,6-Tetra-O-acetyl-alpha-D-glucopyranosyl-fluorid und Benzylalkohol wird in der Gegenwart einer Lewis-Säure wie $BF_3$xEther selektiv das beta-Benzylglucosid hergestellt. Das Produkt wird mit Natriummethylat deacetyliert und anschließend mit einem $C_1$-$C_4$-Aldehyd unter Säurekatalyse in eine 4,6-O-Alkyliden-Verbindung überführt, die letztlich an O-2 und O-3 mittels eines Säureanhydrides oder -halogenides in der Gegenwart einer Base wie Pyridin oder Triethylamin bei -40° C bis 0° C zu einem Benzyl 2,3-di-O-acyl-4,6-O-alkyliden-beta-D-glucopyranosid umgesetzt wird.

Die Herstellung der in der beta-Hydroxy-Form vorliegenden Glucopyranose-Glycosylierungskomponente

kann auf folgende Weise durchgeführt werden: Benzyl 2,3-di-O-acyl-4,6-O-alkyliden-beta-D-glucopyranosid wird in der Gegenwart von Palladium/Kohle oder Palladium/Bariumsulfat und eines Lösungsmittels wie Methanol, Ethanol, Aceton oder Essigsäureethylester oder deren Mischungen, bevorzugt Aceton/Ethanol-Mischungen, bevorzugt unter Normaldruck hydriert, wobei die in der beta-Hydroxy-Form vorliegende Glucopyranose-Komponente quantitativ entsteht.

Die Umsetzung der beta-Hydroxy-Donoren mit einem 4′-O-geschützten 4′-O-Demethyl-4-epi-podophyllotoxin-Derivat der Formel III wird mittels eines Promotors wie $BF_3$xEther oder eines Trifluormethansulfonsäure-tri-($C_1$-$C_4$)-alkylsilylesters in einem wasserfreien organischen Lösungsmittel wie Dichlormethan, Essigsäureethylester, Ether, Aceton oder Acetonitril gegebenenfalls in der Gegenwart eines Trockenmittels wie eines Molekularsiebs bei -30° C bis 0° C durchgeführt. Der Einsatz des Promotors Trifluormethansulfonsäure-tri-methylsilylester ist besonders von Vorteil.

Die Abspaltung der Benzyloxycarbonyl-Schutzgruppe verläuft wie üblich, hydrogenolytisch in der Gegenwart von Palladium/Kohle oder Palladium/Bariumsulfat. Die Abspaltung der Acyl-Schutzgruppen im Kohlenhydrat-Teil und gegebenenfalls am Aglycon der Etoposid-Derivate der Formel I verläuft mit einem basischen Ionenaustauscher, bevorzugt Dowex 1x8 in einem organischen Lösungsmittel wie Methanol, Ethanol, Propanol, Essigsäureethylester, Dichlormethan, Chloroform oder deren Mischungen. Besonders bevorzugt ist die Deacylierung von Chloracetyl-Derivaten der Formel I mittels Ionenaustauscher Dowex 1x8, die in Methanol/Chloroform als Lösungsmittel quantitativ zu Etoposiden ohne die Bildung von Nebenprodukten verläuft.

Acyl-Schutzgruppen sind vor allem Acetyl- oder Mono-, Di- oder Trihalogenacetyl-Schutzgruppen mit Fluor, Chlor oder Brom als Halogen, bevorzugt die Chloracetyl-Schutzgruppe.

Die nachfolgenden Beispiele sollen die Erfindung erläutern, jedoch ohne sie auf die genannten Verbindungen zu beschränken.

## Beispiel 1

Herstellung von Benzyl beta-D-glucosid

Benzyl beta-D-glucopyranosid (Verbindung 1)

5 g (14.2 mmol ) 2,3,4,6-Tetra-O-acetyl-alpha-D-Glucopyranosylfluorid wurden in 50 ml trockenem Acetonitril gelöst und mit 2.2 ml (1.5 eq) Benzylalkohol versetzt. Dem auf 0° C abgekühlten Reaktionsansatz wurden unter Rühren 2.1 ml $Bf_3$xEther zugetropft. Nach 2.5 h (DC: Hexan/Essigsäureethylester) wurde der Reaktionsansatz mit 2.5 ml Triethylamin versetzt und in Vakuum eingeengt. Der in Chloroform gelöste Rückstand wurde mit Phosphat-Puffer, pH 7.5, dann mit gesättigter NaCl-Lösung gewaschen, über Natriumsulfat getrocknet und in Vakuum eingedampft. Das Produkt (6.3 g) wurde in 50 ml trockenem Methanol gelöst, und die Lösung wurde mit Natriummethylat auf pH 11 eingestellt. Nach 4 h wurde mit Dowex 1x8 neutralisiert und in Vakuum eingedampft. Das resultierende Produkt, das mit dem in der Literatur beschriebenen Produkt identisch ($^1$H-NMR, Drehwert) ist, wurde ohne weitere Reinigungsschritte in die nächste Stufe eingesetzt.

## Beispiel 2

Herstellung der 2,3-Di-O-acyl-4,6-ethyliden-glucoside

Benzyl 4,6-O-ethyliden-beta-D-glucopyranosid (Verbindung 2)

58.8 g (157.0 mmol) Verbindung 1 wurden in 600 ml Dioxan suspendiert. Nach der Zugabe von 17.7 ml Acetaldehyd wurde der Ansatz auf 10° C abgekühlt und unter Rühren mit 2 ml konz. $H_2SO_4$ tropfenweise versetzt. Nach 1.5 h wurden weitere 9 ml Acetaldehyd und 0.5 ml konz. $H_2SO_4$ zugegeben. Der Reaktionsansatz wurde dann 12 h bei Raumtemperatur gerührt. Die resultierende rot-braune Lösung wurde mit methanolischer Natriummethylat-Lösung auf pH 7 eingestellt, in Vakuum eingedampft und mit Toluol

nachdestilliert. Der Rückstand wurde in Essigsäureethylester aufgenommen und mit Eiswasser ausgewaschen. Die organische Phase wurde über Natriumsulfat getrocknet und eingedampft. Das in Chloroform/Methanol 7:1 gelöste Produkt wurde über 150 g Kieselgel filtriert, und nach Abdampfen der Lösungsmittel wurde der Rückstand in Ether kristallisiert.
Ausbeute: 52.2 g (81 %), $(alpha)_D^{23}$ = -79.1° (c = 1 in Chloroform)
Schmelzpunkt = 150° C

Benzyl 2,3-di-O-acetyl-4,6-O-ethyliden-beta-D-glucopyranosid (Verbindung 3)

3.51 g (11.8 mmol) Verbindung 2 wurden in 30 ml Dichlormethan und 30 ml Pyridin gelöst und mit 5.3 ml Acetanhydrid bei 0° C versetzt. Nach 12 h Rühren wurde der Reaktionsansatz, wie in der Kohlehydratchemie üblich, aufgearbeitet.
Ausbeute: 5.28 g (100 %), $(alpha)_D^{23}$ = -87° (c = 1 in Chloroform)
Schmelzpunkt = 155° C

Benzyl 2,3-di-O-chloracetyl-4,6-O-ethyliden-beta-D-glucopyranose (Verbindung 4)

25 g (54.3 mmol) Verbindung 2 wurden in 350 ml trockenem Dichlormethan gelöst und mit 56 ml Triethylamin versetzt. Dem auf -20° C abgekühlten Ansatz wurden portionsweise 20 ml Chloracetylchlorid, gelöst in 250 ml Dichlormethan zugegeben. Nach 4 h wurde der Reaktionsansatz erneut mit 11 ml Triethylamin und 3.5 ml Chloracetylchlorid versetzt. Nach weiteren 4 h wurde der Ansatz abfiltriert, und die organische Phase wurde mit eiskaltem Phosphat-Puffer, pH 7.0, ausgewaschen. Die organische Phase wurde über Magnesiumsulfat getrocknet und in Vakuum eingedampft. Der in Chloroform/Essigsäureethylester gelöste Rückstand wurde über 100 g Kieselgel filtriert. Das eingedampfte Produkt kristallisierte aus Chloroform und Petrolether. Ausbeute: 31.0 g (82 %), $(alpha)_D^{23}$ = -54.9° (c = 1 in Dichlormethan)

## Beispiel 3

Herstellung von beta-Hydroxy-Glycosylierungskomponenten

2,3-Di-O-acetyl-4,6-O-ethyliden-beta-D-glucopyranose (Verbindung 5)

1 g (2.6 mmol) Verbindung 3 wurden in 40 ml Essigsäureethylester gelöst und mit 1 g Palladium/Kohle versetzt. Die Reaktionsmischung wurde 1 h bei Raumtemperatur unter Normaldruck hydriert. Nach Abfiltrieren des Katalysators wurde die Lösung in Vakuum bei 35° C abgedampft, und der Rückstand wurde mit Toluol nachdestilliert. Das resultierende Produkt wurde ohne weitere Reinigungsschritte in die nächste Reaktionsstufe eingesetzt. Ausbeute: 0.76 g (99 %), $(alpha)_D^{23}$ = -16.4° (c = 1 in Essigester)

2,3-Di-O-chloracetyl-4,6-O-ethyliden-beta-D-glucopyranose (Verbindung 6)

25 g (55.6 mmol) Verbindung 4 wurden in 600 ml Aceton und 60 ml Ethanol gelöst. Nach der Zugabe von 15 g Palladium/Kohle wurde der Ansatz 1.5 h hydriert. Dann wurde der Katalysator abfiltriert, und die organische Phase wurde bei 35° C abgedampft. Der Rückstand wurde mit Toluol abdestilliert und ohne weitere Reinigungsschritte in die nächste Reaktionsstufe eingesetzt. Ausbeute: 19.8 g (98 %), $(alpha)_D^{23}$ = -19.8° (c = 1 in Aceton)

## Beispiel 4

Glycosylierung von Podophyllotoxin-Derivaten

4′-O-Benzyloxycarbonyl-4-O-(2,3-di-O-chloracetyl-4,6-O-ethyliden- beta-D-glucopyranosyl)-4′-O-demethyl-4-epi-podophyllotoxin (Verbindung 7)

Verfahren A:

15.2 g (42.3 mmol) Verbindung 6, 22.6 g (42.3 mmol) 4′-O-Benzyloxycarbonyl-4′-O-demethyl-4-epi-podophyllotoxin und 20 g Molekularsieb 4 Å wurden in 1000 ml Dichlormethan suspendiert und bei -20° C unter Schutzgas mit 45.5 ml $Bf_3$xEther versetzt. Der Ansatz wurde 17 h bei -20° C gerührt, anschließend mit 46 ml Triethylamin versetzt und abfiltriert. Das Filtrat wurde in Vakuum eingedampft und der Rückstand wurde mit Toluol nachdestilliert. Das resultierende Rohprodukt wurde in Chloroform gelöst und mit Phosphat-Puffer, pH 7.5, anschließend ausgewaschen. Die organische Phase wurde über Natriumsulfat getrocknet und in Vakuum eingedampft. Der Rückstand wurde in einer Lösungsmittelmischung aus Dichlormethan/Petrolether/Aceton 8:2:1 gelöst und über 100 g Kieselgel filtriert. Nach Abdampfen der Lösungsmittel wurde das Produkt aus Chloroform/Petrolether kristallisiert. Ausbeute: 31.8 g (56 %), (alpha)-$_D^{23}$ = -37.9° (c = 1 in Chloroform)

Verfahren B:

15.2 g (42.3 mmol) Verbindung 6, 22.0 g (42.3 mmol) 4′-O-Benzyloxycarbonyl-4′-O-demethyl-4-epi-podophyllotoxin und 20 g Molekularsieb 4 Å wurden in 550 ml Dichlormethan/Aceton 10:1 suspendiert und bei -30° C unter Schutzgas mit 9.4 g Trifluormethansulfonsäuretrimethylsilylester versetzt. Der Reaktionsansatz wurde 6 h bei -25° C gerührt, anschließend mit 50 ml Triethylamin versetzt und abfiltriert. Die weitere Aufarbeitung wurde, wie in Verfahren A beschrieben, durchgeführt. Ausbeute: 34 g (92 %)

4′-O-Benzyloxycarbonyl-4-O-(2,3-di-O-acetyl-4,6-O-ethyliden-beta-D-glucopyranosyl)-4′-O-demethyl-4-epi-podophyllotoxin (Verbindung 8)

1.22 g (4.23 mmol) Verbindung 5, 2.20 g (4.23 mmol) 4′-O-Benzyloxycarbonyl-4′-O-demethyl-4-epi-podophyllotoxin und 2.5 g Molekularsieb 4 Å wurden in 60 ml Dichlormethan/Aceton 10:1 suspendiert und bei -30° C unter Schutzgas mit 0.94 g Trifluormethansulfonsäuretrimethylsilylester versetzt. Nach 8 h Rühren bei -25° C wurde der Reaktionsansatz mit 5 ml Triethylamin versetzt und wie bei der Herstellung der Verbindung 7 aufgearbeitet.
Ausbeute: 2.8 g (82 %)

4′-O-Chloracetyl-4-O-(2,3-di-O-chloracetyl-4,6-O-ethyliden-beta-D-glucopyranosyl)-4′-O-demethyl-4-epi-podophyllotoxin (Verbindung 9)

11 g (30.6 mmol) Verbindung 6, 14.6 g (30.6 mmol) 4′-O-Chloracetyl-4′-O-demethyl-4-epi-podophyllotoxin und 20 g Molekularsieb wurden in 330 ml Dichlormethan/Aceton 10:1 verrührt und bei -30° C mit 6.8 g Trifluormethansulfonsäure-trimethylsilylester versetzt. Nach 7 h wurde der Reaktionsansatz, wie bei der Herstellung von Verbindung 7 beschrieben, aufgearbeitet.
Ausbeute: 21.3 g (86 %)

Beispiel 5

Deacylierung der Etoposid-Vorprodukte

4′-O-Benzyloxycarbonyl-4′-O-demethyl-4-epi-4-O-(4,6-O-ethyliden-beta-D-glucopyranosyl)-podophyllotoxin (Verbindung 10)

27.5 g (31 mmol) Verbindung 7 wurden in 780 ml Dichlormethan und 1225 ml Methanol gelöst. Nach

der Zugabe von 50 g Ionenaustauscher Dowex 1x8 wurde die Reaktionsmischung 1 h bei Raumtemperatur gerührt. Die Suspension wurde abfiltriert, und das Harz wurde mit Methanol nachgewaschen. Nach Eindampfen der Filtrate wurde der Rückstand in Chloroform gelöst, und die Lösung wurde mit Phosphatpuffer, pH 7.5, anschließend mit Wasser ausgewaschen. Die organische Phase wurde über Natriumsulfat getrocknet und in Vakuum eingedampft. Der Rückstand kristallisierte aus Ether/Petrolether.
Ausbeute: 21.5 g (96 %)

4′-O-Demethyl-4-epi-4-O-(4,6-O-ethyliden-beta-D-glucopyranosyl)-podophyllotoxin (Verbindung 11)

20 g (24.6 mmol) Verbindung 9 wurden in 500 ml Dichlormethan und 900 ml Methanol gelöst und mit 50 g Dowex 1x8 versetzt. Die Reaktionsmischung wurde 2 h bei Raumtemperatur gerührt und dann abfiltriert, wobei das Harz mit Methanol nachgewaschen wurde. Die vereinigten Filtrate wurden in Vakuum eingedampft. Der in Dichlormethan aufgenommene Rückstand wurde dann mit Phosphat-Puffer, pH 7.5 und mit Wasser nachgewaschen. Die organische Phase wurde über Natriumsulfat getrocknet und in Vakuum eingedampft. Das Produkt kristallisiert aus Dichlormethan und Hexan.
Ausbeute: 13.6 g (94 %)

Beispiel 6

Hydrogenolytische Abspaltung der Benzyloxycarbonylgruppe

4′-O-Demethyl-4-epi-4-O(4,6-O-ethyliden-beta-D-glucopyranosyl)-podophyllotoxin (Verbindung 11)

9.7 g (13.4 mmol) Verbindung 10 wurden in 260 ml Methanol gelöst und mit 5 g Palladium/Kohle hydriert. Nach 40 min. wurde der Katalysator abfiltriert und die Lösung wurde in Vakuum eingedampft. Der Rückstand wurde in Dichlormethan gelöst und mit Phosphat-Puffer, pH 7.5, anschließend mit Wasser ausgewaschen. Die organische Phase wurde über Natriumsulfat getrocknet und in Vakuum eingedampft. Das Produkt kristallisiert aus Dichlormethan und Hexan.
Ausbeute: 7.25 g ( 92 %)

## Ansprüche

1. Verfahren zur Herstellung eines beta-Glucopyranose-Derivates der Formel I

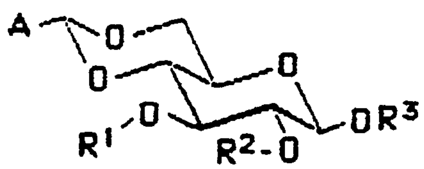

I

worin $R^1$ und $R^2$ H oder eine Acyl-Schutzgruppe und
$R^3$ H, Benzyl oder ein Rest der Formel II

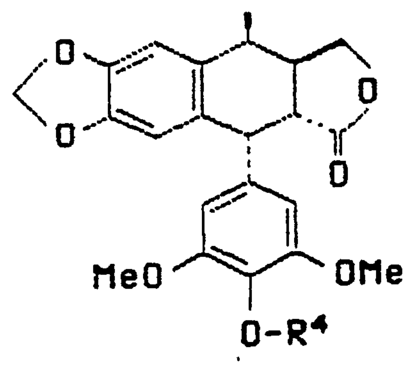

II

mit $R^4$ H oder eine Benzyloxycarbonyl- oder Chloracetyl-Schutzgruppe und A $C_1$-$C_4$-Alkyl darstellt, dadurch gekennzeichnet, daß man in einem Benzylglucopyranosid der Formel I, worin
$R^1$ und $R^2$ eine Acyl-Schutzgruppe,
$R^3$ eine Benzyl-Schutzgruppe und
A $C_1$-$C_4$-Alkyl sind,
die Benzylgruppe hydrogenolytisch in der Gegenwart eines Hydrierungskatalysators und eines organischen Lösungsmittels abspaltet, wobei ein in der beta-Hydroxy-Form vorliegendes Glucopyranose-Derivat der Formel I, worin $R^1$, $R^2$ und A ihre Bedeutung beibehalten und $R^3$ ein Wasserstoffatom ist, gebildet wird, anschließend diese Glycosidierungskomponente mit einem epi-Podophyllotoxin-Derivat der Formel III

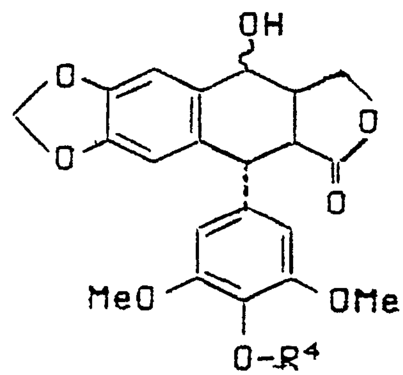

III

worin
$R^4$ Benzyloxycarbonyl- oder Chloracetyl-Schutzgruppe ist, in der Gegenwart eines Promotors wie $BF_3$xEther oder eines Trifluormethansulfonsäure-tri-($C_1$-$C_4$)alkylsilylesters und eines organischen Lösungsmittels, gegebenenfalls in der Präsenz eines Trockenmittels bei -30° C bis 0° C umsetzt, wobei ein beta-Glycosid der Formel I, worin $R^1$, $R^2$, $R^4$ und A ihre Bedeutung beibehalten und $R^3$ einen Rest der Formel II darstellt, entsteht, und in einem gebildeten Produkt die Benzyloxycarbonyl-Schutzgruppe hydrogenolytisch und die Acyl-Schutzgruppe mittels basischer Ionenaustauscher in der Gegenwart eines polaren Lösungmittels abspaltet, wobei ein Produkt der Formel I entsteht, worin
$R^1$ und $R^2$ Wasserstoff,
$R^3$ ein Rest der Formel II mit $R^4$ gleich Wasserstoff und
A $C_1$-$C_4$-Alkyl sind.

2. Verfahren nach Anspruch 1, worin
$R^1$ und $R^2$ H oder die Acetyl- oder Chloracetyl-Schutzgruppe
$R^3$ H, oder Benzyl oder ein Rest der Formel II mit
$R^4$ H oder eine Benzyloxycarbonyl- oder Chloracetyl-Schutzgruppe und
A Methyl darstellen.

3. Verfahren zur Herstellung eines beta-Hydroxy-Glucopyranose-Derivates der Formel I nach Anspruch 1, worin
$R^1$ und $R^2$ eine Acyl-Schutzgruppe,
$R^3$ Wasserstoffatom und
A $C_1$-$C_4$-Alkyl darstellen,

dadurch gekennzeichnet, daß man ein beta-Benzylglucosid der Formel I, worin

$R^1$ und $R^2$ eine Acyl-Schutzgruppe,

$R^3$ Benzyl und

A $C_1$-$C_4$-Alkyl sind,

in der Gegenwart eines Hydrierungsmittels wie Palladium/Kohle oder Palladium/Bariumsulfat in einem organischen Lösungsmittel wie Methanol, Ethanol, Aceton, Essigsäureethylester oder deren Mischungen, bevorzugt Aceton/Ethanol-Mischungen hydriert, wobei ein in der beta-Hydroxy-Form vorliegendes Produkt der Formel I gebildet wird, worin

$R^1$, $R^2$ und A unverändert sind und

R ein Wasserstoffatom ist.

4. Verwendung einer nach Anspruch 3 hergestellten Verbindung in einem Verfahren zur Herstellung von Etoposiden, dadurch gekennzeichnet, daß man diese Verbindung mit einem 4′-O- geschützten 4′-O-Demethyl-4-epi-podophyllotoxin mittels eines Promotors wie $BF_3$xEther oder Trifluormethansulfonsäure-tri-($C_1$-$C_4$)alkylsilylester in einem wasserfreien organischen Lösungsmittel, gegebenenfalls in der Gegenwart eines Trockenmittels wie eines Molekularsiebs bei -30° C bis 0° C zu einem beta- Glycosid der Formel I umsetzt, wobei

$R^1$ und $R^2$ Acyl-Schutzgruppe,

$R^3$ ein epi-Podophyllotoxin-Rest der Formel II mit

$R^4$ Benzyloxycarbonyl- oder eine Acyl-Schutzgruppe und

A $C_1$-$C_4$-Alkyl sind.

5. Verwendung nach Anspruch 4 dadurch gekennzeichnet, daß in dem Verfahren ein Trifluormethansulfonsäure tri-($C_1$-$C_4$)-alkylsilylester verwendet wird.

6. Verfahren zur Deacylierung eines 4-O-Glucosyl-epi-podophyllotoxin-Derivates der Formel I in Anspruch 1, dadurch gekennzeichnet, daß man eine acylierte Verbindung mit einem basischen Ionenaustauscher in einem Lösungsmittel wie Methanol, Ethanol, Propanol, Essigsäuremethylester, Dichlormethan, Chloroform oder deren Mischungen, bevorzugt mit einer Methanol/Chloroform-Mischung behandelt, wobei eine Verbindung der Formel I entsteht, worin die Reste

$R^1$, $R^2$ und $R^4$ Wasserstoff,

$R^3$ einen epi-Podophyllotoxin-Rest der Formel II und

A ($C_1$-$C_4$)-Alkyl darstellen.

7. Verfahren nach Anspruch 6, dadurch gekennzeichnet, daß als Ionenaustauscher bevorzugt Dowex 1x8 verwendet wird.

Europäisches Patentamt

**EUROPÄISCHER RECHERCHENBERICHT**

Nummer der Anmeldung

EP 90107637.2

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int Cl 5) |
|---|---|---|---|
| X | EP - A2/A3 - 0 162 701 (NIPPON) * Ansprüche; Seiten 2,3 (insbes. Seite 3, erster Absatz), 6,7; Beispiele 15,17 * | 1-5 | C 07 H 17/04<br>C 07 H 15/06 |
| A | CHEMISTRY LETTERS, Nr. 1, 1987 H. SAITO et al. "Syntheses of all four possible diastereomers of etoposide and its aminoglycosidic analogues have been achieved via optical resolution of (+)-podophyllotoxin by glycosidation with D- and L-sugars." Seiten 799-802 * Gesamt * | 1-5 | |
| D,A | EP - A2/A3 - 0 226 202 (BAR-ILAN UNIVERSITY) * Beispiele; Ansprüche * | 1-6 | RECHERCHIERTE SACHGEBIETE (Int Cl 5) |
| D,A | EP - A1 - 0 111 058 (NIPPON) * Beispiele; Ansprüche * | 1-6 | C 07 H 17/00<br>C 07 H 15/00 |

Der vorliegende Recherchenbericht wurde für alle Patentansprüche erstellt.

| Recherchenort | Abschlußdatum der Recherche | Prüfer |
|---|---|---|
| WIEN | 25-07-1990 | JANISCH |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82